(19) **Europäisches Patentamt / European Patent Office / Office européen des brevets**

(11) **EP 3 939 051 B1**

(12) **EUROPEAN PATENT SPECIFICATION**

(45) Date of publication and mention
of the grant of the patent:
**12.03.2025 Bulletin 2025/11**

(21) Application number: **20711027.1**

(22) Date of filing: **13.03.2020**

(51) International Patent Classification (IPC):
**G16H 30/20** *(2018.01)*    **G16H 30/40** *(2018.01)*
**G16H 40/63** *(2018.01)*

(52) Cooperative Patent Classification (CPC):
**G16H 30/20; G16H 30/40; G16H 40/63**

(86) International application number:
**PCT/IB2020/052272**

(87) International publication number:
**WO 2020/188428 (24.09.2020 Gazette 2020/39)**

(54) **SYSTEM AND COMPUTER IMPLEMENTED METHOD FOR 3D PROCESSING OF A TOMOGRAPHY TEST**

SYSTEM UND COMPUTERIMPLEMENTIERTES VERFAHREN ZUR 3D-VERARBEITUNG EINES TOMOGRAFIETESTS

SYSTÈME ET PROCÉDÉ EXÉCUTÉ PAR ORDINATEUR POUR LE TRAITEMENT 3D D'UN EXAMEN TOMOGRAPHIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.03.2019 IT 201900003809**

(43) Date of publication of application:
**19.01.2022 Bulletin 2022/03**

(73) Proprietor: **Witapp S.r.l.**
**50127 Firenze (FI) (IT)**

(72) Inventors:
• **CHECCACCI, Iacopo**
**50124 Firenze (IT)**
• **LAPENTA, Giuseppe**
**85047 Moliterno (potenza) (IT)**

(74) Representative: **Penza, Giancarlo**
**Bugnion S.p.A.**
**Viale Lancetti, 17**
**20158 Milano (IT)**

(56) References cited:
**US-A1- 2013 044 856    US-A1- 2018 165 867**

• **MORALES MOJICA CRISTINA MARIE ET AL: "Holographic Interface for three-dimensional Visualization of MRI on HoloLens: A Prototype Platform for MRI Guided Neurosurgeries", 2017 IEEE 17TH INTERNATIONAL CONFERENCE ON BIOINFORMATICS AND BIOENGINEERING (BIBE), IEEE, 23 October 2017 (2017-10-23), pages 21 - 27, XP033293739, DOI: 10.1109/ BIBE.2017.00-84**

## Description

FIELD OF APPLICATION

The present invention relates to a system for 3D processing of a tomography test.

The present invention further relates to a computer-implemented method for 3D processing of a tomography test.

In particular, the invention relates to a system/-method for 3D processing of a tomography test wherein the tomography test comprises generating a file of output data comprising patient data and image data, and the following description makes reference to this field of application in order to simply the disclosure.

PRIOR ART

Within hospitals, and in particular in a context of the admission of a patient in emergency conditions, at the moment the procedure whereby information of a tomography test performed on the patient is transferred to a doctor, in particular a surgeon - who requests the test itself under conditions of urgency - takes place by recording the test images on a compact disk; such information is subsequently viewed by the requesting doctor by means of suitable viewing software.

The need that is most greatly felt by a surgeon while planning an operation, i.e. in the pre-operative phase, is to have a view of the patient's tissues or organs that the operation must be performed on which is as detailed and complete as possible; a three-dimensional representation makes it possible to accurately reproduce the state of the area in which it will be necessary to operate and thus to plan the operation in optimal fashion.

Unfortunately, the software presently available to hospitals for this purpose enables the consultation of static, non-interactive information/images that oblige the surgeon who is planning the operation to ask the tomography technician, in particular a radiologist, for more precise details on the effects/movements of the parts involved; such details are usually provided by means of a video prepared ad hoc by the tomography technician.

A critical issue in this procedure is represented by the time it takes for the creation of a 3D tomography model and rendering of the video, which occupy the radiologist's activity for an average time of 25 minutes, excluding further requests for modifications/additions made by the surgeon after viewing the model or due to errors arising from incomprehension between the parties.

A second critical issue is represented by the fact that the video created with a three-dimensional model is viewed by means of two-dimensional media such as a computer monitor, which does not give a perception of space and does not maintain the proportion of the organ to be operated on.

The procedure described thus poses 3 main problems:

- Static processing of the 3D view
- Lack of perception of the real dimensions, in particular the depth of the element being viewed
- Lengthy information transfer times

Added to these problems there is the critical issue of passing tomography data/information to the members of a medical staff so that each one may have an optimal view of the part to which the greatest attention must be paid during the operation.

The general object of the present invention is to overcome the drawbacks of the prior art described.

A specific object of the invention is to provide a system for 3D processing of a tomography test that is optimised in terms of efficiency of execution.

Another object of the invention is to provide a system for 3D processing of a tomography test that enables the surgeon to customise the 3D view directly and in real time.

A further object of the invention is to provide a system for 3D processing of a tomography test which enables tomographic images to be directed, in a targeted manner, to different members of a surgical staff prior to an operation.

MORALES MOJICA CRISTINA MARIE ET AL: "Holographic Interface for three-dimensional Visualization of MRI on HoloLens: A Prototype Platform for MRI Guided Neurosurgeries",2017 IEEE 17TH INTERNATIONAL CONFERENCE ON BIOINFORMATICS AND BIOENGINEERING (BIBE), IEEE, 23 October 2017 discloses a system for holographic visualization of high-resolution 3D MRI data which offers an intuitive and interactive perspective of the complex brain vasculature and anatomical structures based on rendering of a mesh structure. However, the system shown in this document is not structured in hardware and software to ensure compatibility between the data sent and the viewer prepared, but only to ensure archiving of the data sent. Similar approaches can also be found in US 2013/044856 A1 and US 2018/165867 A1, however both documents also do not suggest this feature.

SUMMARY OF THE INVENTION

The invention is defined by the appended claims.

In a first aspect of the invention, these and other objects are achieved by a computer-implemented method for 3D processing of a tomography test, according to what is claimed in claim 1.

In a second aspect of the invention, these and other objects are achieved by a system for 3D processing of a tomography test according to what is claimed in claim 12.

Further advantages are described in the dependent claims.

The invention achieves the following technical effects:

- efficiency of execution with consequent reduced times of analysis by surgeons, especially under emergency presurgical conditions;
- in particular, direct, real-time interaction is achieved between surgeons and the 3D view based on the specific planning needs of an urgent operation or needs arising spontaneously in the presurgical phase, with the advantage of not requiring subsequent video processing by the tomography operator;
- customisation of the 3D view;
- optimisation of the distribution of images to the specific doctors who perform the operation.

The aforementioned technical effects/advantages and other technical effects/advantages of the invention will emerge in greater detail from the following description of an example embodiment, given by way of illustration and not by way of limitation with reference to the appended drawings.

BRIEF DESCRIPTION OF THE DRAWINGS

Figure 1 is a block diagram of the system/method according to the invention, in an overall view.

Figure 2 is a detail of the data transmission in the system/method of figure 1.

Figure 3 is a detail of the transmission protocol in the system/method of figure 1.

Figure 4 is a schematic representation of an integrated viewing station comprised in the system according to the invention.

DETAILED DESCRIPTION

The design presented aims to improve diagnostic times for operators in the health sector by using digital diagnostic imaging solutions based on edge computing technologies.

The solution is capable of offering greater efficiency within hospitals and private clinics thanks to an improvement in performance, with a faster processing of images and more rapid use of the same, which may be used in planning an operation, i.e. in the presurgical phase.

The solution is mainly composed of a system of HW/SW platforms comprising software for generating computed tomography images, software for processing the images generated, software for adapting the images to enable an optimal view aimed at a specific user, and a mixed reality viewer for viewing the images adapted for the specific user.

The invention comprises performing a tomography test E_TAC on a patient P and generating a corresponding file of output data TAC_out in a first predefined format for tomography tests F_TAC.

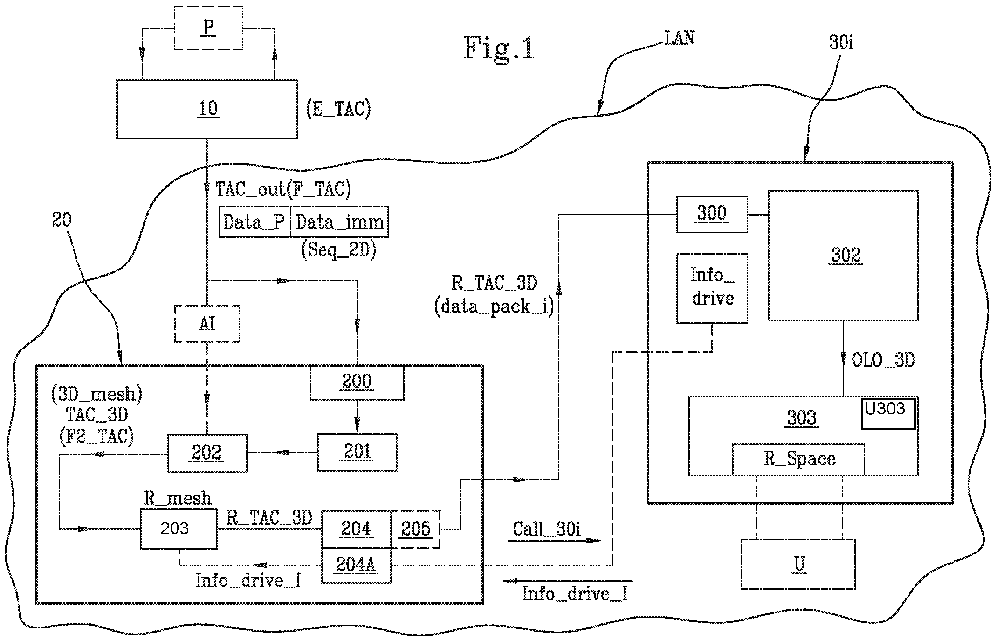

With particular reference to figure 1, a system for 3D processing of a tomography test E_TAC comprises a first processing station 10 configured to perform a tomography test E_TAC on a patient P.

The first processing station 10 preferably comprises a computed tomography machine.

The first predefined format for tomography tests F_TAC is preferably the "DICOM" format.

In addition to the actual image, the DICOM file also includes a header. Various information is contained in the DICOM header, for example: patient's name and surname, type of scan, image position and size, etc.

The size of the header will obviously vary according to the amount of information stored. All of the information stored in the header is catalogued into groups of elements, also known as "DICOM tags".

The output file TAC_out comprises one or more from among, at least, patient data Data_P and patient image data Data_imm comprising a sequence of two-dimensional slices seq_2D representative of an internal section of the patient P.

The invention comprises performing software processing of the patient data Data_P and the patient image data Data_Imm.

In a preferred embodiment of the invention, with reference to figure 1, the system according to the invention comprises a second processing station 20, in particular an electronic digital processor of medical images, preferably CAT images, configured for that purpose.

In the course of the present description and in the subsequent claims, the second processing station 20 will be described as logically divided into distinct functional modules - storage modules or operating modules - which perform specific functions.

The second processing station 20 can consist of a single electronic device, appropriately programmed to perform the functions described, and the different modules can correspond to hardware entities and/or routine software belonging to the programmed device.

Alternatively, or in addition, such functions can be performed by a plurality of electronic devices over which the aforesaid functional modules can be distributed.

The second processing station 20, moreover, can avail itself of one or more processors for executing the instructions contained in the storage modules.

Moreover, the aforesaid functional modules can be distributed over different local or remote computers based on the architecture of the network they reside in.

With reference to figure 1, the second processing station 20 is configured to receive the output file TAC_out.

The second processing station 20 comprises a first receiving module 200 configured for this function.

The second processing station 20 is further configured graphically process the sequence of two-dimensional slices seq_2D.

The second processing station 20 comprises a processing module 201 configured for this function.

The second processing station is configured to generate a 3D file TAC_3D in a second predefined format for tomography tests F2_TAC as a function of the result of the processing.

The second predefined format for tomography tests F2_TAC is preferably an STL file format.

In particular, after the loading of the output file TAC_OUT, in particular in DICOM format, it is envisaged that one or more tissues of the patient may be selected so as to obtain an overall 3D representation depicting different tissues highlighted in different ways, in particular distinguished by different colours.

For this purpose, distinct 3D models are provided in a combined view, i.e. overlaid; the 3D file TAC_3D comprises the models treated like a single overall 3D model.

In other words, the generated 3D file TAC_3D comprises the representation of the one or more tissues of the patient highlighted in different ways, in particular distinguished by different colours, in a combined view.

Therefore, the processing step comprises processing distinct 3D models provided in a combined view in the 3D file TAC_3D, so that the overall 3D file TAC_3D represents various tissues of the patient highlighted in different ways, in particular distinguished by different colours.

The processing module 201 is configured for this purpose.

The technical effect is to render the tissues clearly distinguishable from one another.

The 3D model comprising the 3D representations of tissues rendered in different ways can be sent as a single object to the application at the integrated viewing station 30i, in particular to the viewer 303. Via the integrated viewing station 30i, it is possible, by means of the viewer 303, to consult the 3D hologram comprising the tissues in the established colours. The viewer 303 is further configured to enable or disable the viewing of every single level of tissue.

The technical effect achieved is the viewing, by the user, of the hologram of all tissues simultaneously or only the selected ones.

The generated 3D file TAC_3D comprises a plurality of 3D cells 3D_cell that determine a 3D mesh-like structure 3D_mesh of the patient image data Data_Imm.

With reference to figure 1, the second processing station 20 comprises a 3D calculating module 202 configured to produce the 3D file TAC_3D as previously described.

In a second embodiment of the invention, the software processing of the patient data Data_P and/or the patient image data Data_Imm and/or the one or more tissues of the patient highlighted in different ways is performed by applying a neural network system AI con-

figured to generate the 3D file TAC_3D from the output file TAC_out generated by the first processing station 10.

In particular, the neural network is a convolutional neural network.

The network is trained to recognise, in an input tomography test (in particular in the DICOM format, 512x512 pixels), i.e. based on the output file TAC_out generated by the first processing station 10, which areas correspond to different tissues or organs of interest.

The computational model thus produces a semantic segmentation of the input tomography test which assigns a specific class, such as bone, vessel, and other classes of tissues and/or organs, to every pixel identified.

During training, every input tomography test is accompanied by a corresponding reference segmentation, in particular prepared by an expert.

The dataset thus constructed is increased during training through ad hoc rotations and deformations.

The calibration of the network parameters is guided by stochastic gradient descent and has the objective of minimising a measure of error, at the level of individual pixels, between the output of the network and the desired reference segmentation.

A certain number of tomographic tests, not observed by the network during training, is used as the validation set to interrupt the gradient descent and the consequent updating of the network parameters.

The second processing station 20 is further configured to determine a mesh reduction factor R_mesh for the 3D file TAC_3D, thus determining a reduced 3D file R_TAC_3D.

With reference to figure 1, the second processing station 20 comprises a reduction module 203 configured to determine the mesh reduction factor R_mesh.

The second processing station 20 further envisages transmitting the reduced 3D file R_TAC_3D conditionally in a local network LAN.

With reference to figure 1, the second processing station 20 comprises a conditioned transmission module 204 configured for this function.

The invention further comprises providing at least one integrated viewing station 30i, i=1..n in the local network LAN.

A plurality of integrated viewing stations 30i can be provided, each with specific viewing features that can be customised according to the part of the patient to be shown during an operation.

Every integrated viewing station 30i comprises identification, activation and configuration information info_drive characteristic of the integrated viewing station 30i, which enable an optimisation of the work of the surgeon using the specific station.

With reference to figure 1, the information in the integrated viewing station 30i is comprised in a storage module 301 .

Consequently, the local network LAN according to the invention envisages the presence of a plurality of integrated viewing stations 30i configured to cooperate, but with different predefined functions.

At least one integrated viewing station 30i preferably comprises a viewer 303, in particular a mixed reality viewer.

One example of a standard integrated viewing station, i.e. without any customisation or possibility of selecting the input images, is Microsoft HoloLens.

According to the invention, there is provided a plurality of mixed reality viewers 303 associated with corresponding integrated viewing stations 30i. The provision of specific viewers 303 enables optimised viewing of various anatomical features that can also be analysed during a presurgical phase. For example, for every region of the patient's body having a more complex anatomical structure, a viewer with extremely high resolution might be necessary, whereas in the case of a less complex region, a viewer with lower resolution but a greater computing power might be sufficient.

Balancing the features of the viewers enables the technical effect of resource processing optimisation.

Consequently, as will be better detailed further below in the description, the mesh reduction factor will be optimised also as a function of the features of the viewer 303 used, in particular a mixed reality viewer.

Preferably, a selection interface 205 is provided which is configured to select an integrated viewing station 30i as a function of the features of the viewer 303.

According to the invention, the conditioned transmission over the local network LAN is carried out by the conditioned transmission module 204, which comprises an identification module 204A configured to carry out the described identification functions.

The conditioned transmission comprises sending call signals Call_i in the local network LAN in order to identify an integrated viewing station 30i previously configured to receive the 3D file TAC_3D, detect the identification, activation and configuration information info_drive_I comprised in the storage module 301 of the integrated viewing station 30i identified, and send the identification, activation and configuration information info_drive_I detected to the reduction module 203.

The integrated viewing station 30i has preferably been previously configured by means of the selection interface 205.

The technical effect achieved is a viewing of the 3D image information on the viewer which is as reliable as possible, given the complete compatibility between the 3D data generated and the technical graphic processing features of the viewer 303.

It is fundamental, in fact, in the presurgical phase and particularly under conditions of urgency, that the images do not show artifacts or distortions caused by an incomplete conformity of the input data with the data processable by the viewer 303.

According to the invention, the mesh reduction factor (mesh) R_mesh for the integrated viewing station 30i identified is calculated, in particular by the reduction module 203, based on the identification, activation and configuration information info_drive_I received from the conditioned transmission module 204.

Specifically, the algorithm for calculating the mesh reduction ALG_R_mesh comprises calculating the mesh reduction factor R_mesh starting from an estimated size ES of the output 3D file TAC_3D and the maximum size that the file can reach in order to be sustainable for the process of importing the mesh to the integrated viewing station 30i, in particular a HoloLens proprietary app.

The latter information is contained in the information info_drive of every integrated viewing station 30i.

According to the invention, the mesh reduction factor R_mesh is the percentage of reduction to be applied to the mesh in order to be imported by the integrated viewing station 30i, in particular the HoloLens proprietary app.

According to the invention, the following relation applies:

$$R\_mesh = 1 - (MFS / ES)$$

with

k = 180
ES = k * CN / 1000000
wherein:

    k: constant
    CN(Cell Number): is the number of cells of the 3D model obtained.
    ES(Estimated Size): is the estimated size of the *stl* file.
    MFS(Max File Size) is the maximum size that the file can reach in order to be sustainable for the process of importing the mesh to the integrated viewing station 30i, in particular the HoloLens proprietary app.

Furthermore, according to the invention, the mesh reduction factor R_mesh is further defined as a function of the viewer 303 identified.

The process of creating the 3D model is thus capable of adapting the result to the input data, i.e. the estimated size ES of the output 3D file TAC_3D, and the capabilities of the device in which the output will be viewed, i.e. the maximum size that the file can reach in order to be sustainable for the process of importing the mesh to the integrated viewing station 30i. This process makes it possible always to have a useful and usable result, in the integrated viewing station 30i, of the holographic representation of the input CT test, i.e. of the output 3D file TAC___3D.

The transmission of the three-dimensional model to the integrated viewing station 30i is carried out with a specific method.

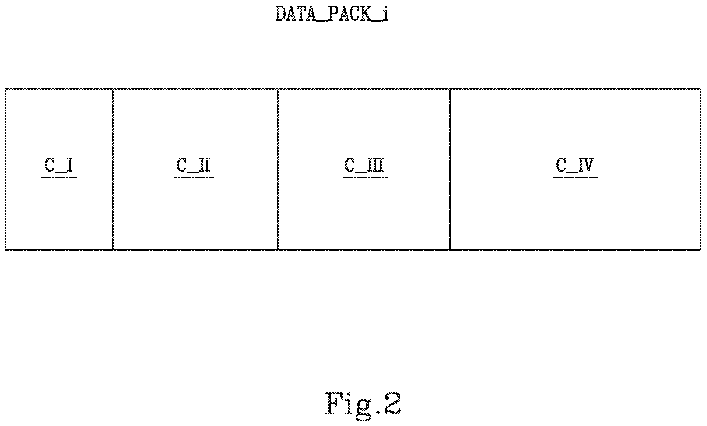

With particular reference to figure 2, the conditioned transmission module 204 is configured to transmit, in the local network LAN, the reduced 3D file R_TAC_3D to the integrated viewing station 30i identified through a data pack data pack_I comprising:

- an initialisation field C_I;
- a message field C_II, in particular comprising messages for and from the viewer to handle the sending of data;
- a size field C_III comprising the size of the data segment to be sent;
- a data field C_IV comprising the data to be sent - the 3D images - of the size indicated in the size field C_III;

According to the invention, the size field C_III is defined as a function of the identification, activation and configuration information info_drive received.

In other words, the data pack data pack_I varies dynamically as a function of the features of the integrated viewing station 30i.

In other words, a dedicated transmission protocol has been implemented whereby it is possible to send, via the network, the 3D model, i.e. the 3D file R_TAC_3D, including information regarding the structure and composition thereof info_drive, to the integrated viewing station 30i, i.e. to the application for the mixed reality viewer 303, which receives said model and information in order to be able to load it and view it dynamically, so that it need not be preloaded into the integrated viewing station 30i itself.

It is possible, therefore, to dynamically identify the mixed reality viewers 303 over the same network in which the integrated viewing station 30i for the mixed reality viewer 303 is active. This makes it possible not to have to pre-configure the viewers 303 and to be able to use more than one at any time.

The technical effect achieved is an optimal correspondence between the data sent and the user prepared to receive it; in the presurgical phase, in particular under conditions of urgency, the distribution of precise information to the various surgeons prepared to receive and view different information for the same operation can be crucial for the success of the operation.

With reference to figure 2, the data structure for handling the sending of the three-dimensional model from the second processing station 20 to the integrated viewing station 30i, in an example embodiment, can be described as follows:

C_I = initialisation byte: it is used to check that the pack actually belongs to the app;

C_II = message segment: UTF-8 encoding of a message with a maximum of 32 characters, which will then be interpreted by the client or the server;

C_III = Data length segment: UTF-8 encoding of the size of the data segment, the number can have a maximum of 32 digits;

C_IV = data segment: it contains the data that are about to be sent and can have a maximum length of 65536 bytes. The variable length of this segment makes it possible to send small or large-sized files:

- if the file is smaller in size than the maximum length, the data segment will be smaller.
- if the file is larger in size, it will be divided into several packs and the data segment will be of the maximum size.

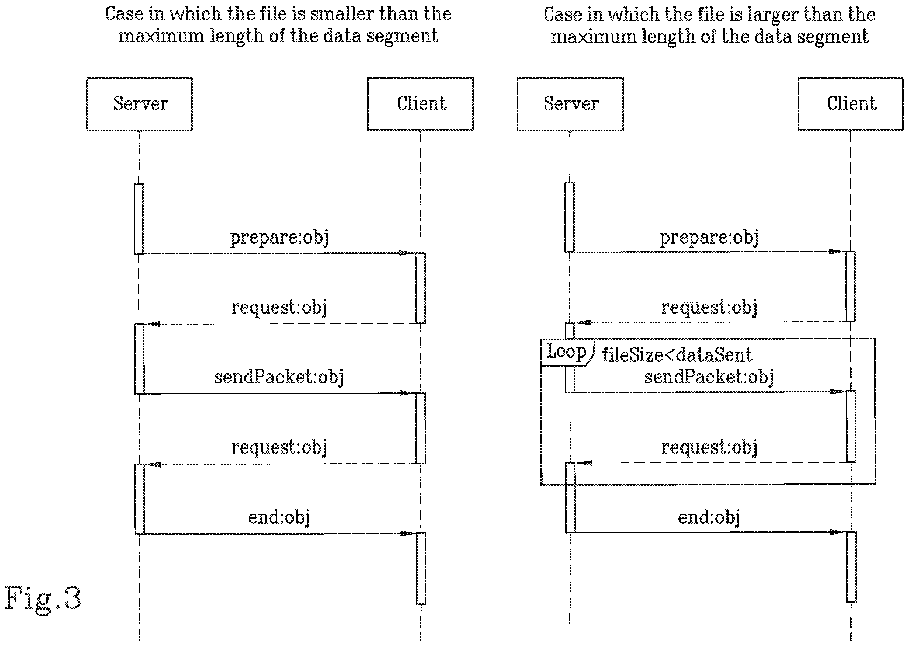

Based on the dimensions of the 3D data file R_TAC_3D, the transmission takes place, for example, as shown in figure 3, wherein:

- "prepare:obj" is a server request to the client to prepare to receive the file
- "request:obj" is a client response to the server to say that it is ready to receive more data
- "sendPacket:obj" is a server message to the client which indicates that the current pack contains data of the file
- "end:obj" is a server message to the client which notifies the client that all the data of the file have been sent.

With particular reference to figure 1, the integrated viewing station 30i in the local network LAN is configured to receive the reduced 3D file R_TAC_3D transmitted by the second processing station 20.

The invention envisages that the integrated viewing station 30i receives the reduced 3D file R_TAC_3D transmitted by the second processing station 20.

The integrated viewing station 30i comprises a second receiving module 300 configured for that function.

The invention further comprises determining a 3D hologram graphic model OLO_3D of the patient image data Data_Imm as a function of the reduced 3D file R_TAC_3D received.

The integrated viewing station 30i comprises a processing unit 302 configured to determine the 3D hologram graphic model 3D OLO_3D of the patient image data Data_Imm as a function of the reduced 3D file R_TAC_3D.

In particular, a step of selecting one or more tissues of the patient from the reduced 3D file R_TAC_3D is envisaged.

The integrated viewing station 30i comprises a selection interface U303 configured to enable the selection of one or more tissues of the patient from the reduced 3D file R_TAC_3D generated.

Through the integrated viewing station 30i, it is thus possible, via the selection interface U303 of the viewer 303, to consult the 3D hologram comprising the tissues of the patient in the established colours.

The viewer 303 is further configured to enable or disable viewing of every single level of tissue of the patient, by acting through the selection interface U303.

The technical effect achieved is the viewing, by the user, of the hologram of all tissues simultaneously or only the selected ones.

As already mentioned, the invention comprises configuring a viewer 303 to interact with a user U and to represent the 3D hologram OLO_3D in a real viewing space R_space.

As said previously, the configuration of specific viewers 303 enables an optimised view of different anatomical features, which may be analysed in the presurgical phase.

The viewer 303 is configured to perform the interaction between the user U and the 3D hologram graphic model OLO_3D by implementing one or more functions from among movement, rotation and enlargement of the 3D hologram graphic model OLO_3D.

The interactions on the 3D model from the mixed reality viewer 303 are implemented by exploiting the gestures of the viewer itself, but with the implementation of the dedicated functions, since the native interface of the viewer provides the basic software components, but does not implement the functions thereof, unlike what is described in the present invention. Therefore, though the interface of the mixed reality viewer 303 is exploited in order to capture the input, i.e. the 3D hologram OLO_3D, the interactions on the 3D model are the result of the implementation taking place on the integrated viewing station 30i, i.e. on the application for the viewer 303, according to the invention.

According to the invention, the movement, rotation and enlargement functions comprise the actions of:

- detection of a hand M of the user U;
- selection gestures with the hand M of the user U;
- detection of a movement of the hand M in the selection status;
- detection of a movement of two hands M in the selection status.

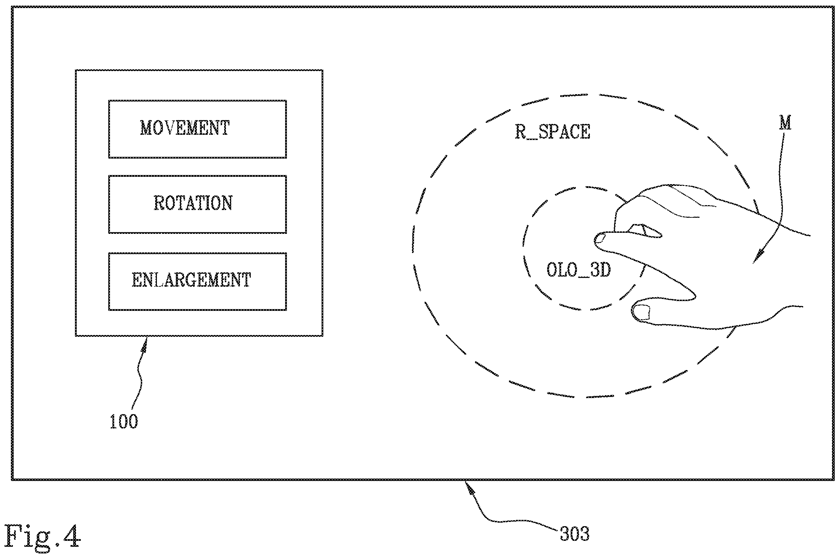

In other words, with particular reference to figure 4, the integrated viewing station 30i, in particular the HoloLens proprietary application, implements the movement, rotation and enlargement operations (menu 100) as follows:

Movement: after having selected the movement action from the specific menu 100, the user U focuses on the hologram OLO_3D using the system gaze function, puts his/her hand M in front of him/herself using the system's click gesture, and moves his/her hand M. The hologram OLO_3D moves according to the movement of the hand M of the user, based on the position of the hand M in the space R_space, and applying its movement to the 3D model.

Rotation: after having selected the rotation action from the specific menu, the user focuses on the hologram OLO_3D using the system gaze function, puts his/her hand M in front of him/herself using the system's click gesture, and moves his/her hand M. The hologram OLO_3D rotates in the direction in which the hand M of the user has moved, at a speed that is directly proportional to the speed of the hand M.

The rotation applied to the hologram OLO_3D is calculated by measuring the distance between the user U - and thus the integrated viewing station 30i - and the hand M in every frame, so as to be able to apply a rotation to the hologram OLO_3D that is consistent with the direction and speed of the gesture, irrespective of the position of the user U relative to the hologram OLO_3D itself.

Enlargement: after having selected the enlargement action from the specific menu 100, the user focuses on the hologram OLO_3D using the system gaze function, puts two hands M in front of him/herself using the system's click gesture, and moves them closer to or farther from each other. The hologram is reduced in size when the hands are moved nearer, and increased in size when they are moved farther.

The hologram size modification factor is calculated by adding to its current size the difference between the distance between the hands at the beginning of the operation and that at the subsequent frame, so that the speed in the variation of the size of the hologram varies in a manner that is directly proportional to the speed of movement of the hands.

The invention thus comprises calculating the mesh reduction factor R_mesh of the reduced 3D file R_TAC_3D as a function of the activation and configuration information info_drive, thus ensuring complete compatibility of the viewer 303 with the 3D hologram graphic model OLO_3D generated and allowing the user U to interact interactively with the 3D hologram graphic model 3D OLO_3D generated.

In other words, the reduced 3D file R_TAC_3D has a mesh reduction factor R_mesh calculated as a function of said activation and configuration information info_drive_i, thus ensuring complete compatibility of the viewer 303 with the 3D hologram graphic model OLO_3D generated and allowing the user U to interact interactively with the 3D hologram graphic model 3D OLO_3D generated.

The invention as described is implemented in an aspect thereof as a computer-implemented method for 3D processing of a tomography test E_TAC comprising a plurality of operating steps whereby the functions of the components described are performed.

A method and a system for 3D processing of a tomography test has been presented which achieves the following technical effects:

- efficiency of execution, with a consequent reduction in the times of analysis by surgeons, especially under emergency conditions;
- in particular, direct, real-time interaction is achieved between surgeons and the 3D view based on the specific needs for planning an urgent operation, i.e. in the presurgical phase, with the advantage of not requiring subsequent video processing on the part of the tomography technician;

- customisation of the 3D view;
- optimisation of the distribution of the images to specific doctors in the presurgical phase.

**Claims**

1. Computer-implemented method for 3D processing of a tomography test (E_TAC) comprising the steps of:

    performing a tomography test (E_TAC) on a patient (P) and generating a corresponding file of output data (TAC_out) in a first predefined format for tomography tests (F_TAC), wherein said output file (TAC_out) comprises one or more from among, at least:

        - patient data (Data_P);
        - patient image data (Data_imm) comprising a sequence of two-dimensional slices (seq_2D) representative of an internal section of the patient (P);

    performing a software processing of said data (Data_P,Data_Imm) to generate a 3D file (TAC_3D) in a second predefined format for tomography tests (F2_TAC) as a function of said output file (TAC_out) generated, wherein said 3D file (TAC_3D) comprises a plurality of 3D cells (3D_cell) that determine a 3D mesh-like structure (3D_mesh) of said image data of the patient (Data_Imm);

        - determining a mesh reduction factor (R_mesh) for said 3D file (TAC_3D), thus determining a reduced 3D file (R_TAC_3D);

    wherein an algorithm for calculating the mesh reduction (ALG_R_mesh) comprises calculating the mesh reduction factor (R_mesh) starting from an estimated size (ES) of said output 3D file (TAC_3D) and the maximum size that the file can reach in order to be sustainable for the process of importing the mesh to an integrated viewing station (30i);

        - transmitting said reduced 3D file (R_TAC_3D) conditionally in a local network (LAN);

    wherein said step of transmitting provides transmitting in said local network (LAN) said 3D file (TAC_3D) to said identified integrated viewing station (30i) through a data pack comprising:

    - an initialisation field (C_I);
    - a message field (C_II), in particular comprising messages for and from the viewer to handle the sending of data
    - a size field (C_III) comprising the size of the data segment to be sent;
    - a data field (C_IV) comprising the size data indicated in the size field (III) to be sent;

wherein said size field (C_III) is defined as a function of said identification,
activation and configuration information (info_drive) received.
wherein the method further comprises the step of:

    providing, in said local network (LAN), an integrated viewing station (30i) comprising identification, activation and configuration information (info_drive) characteristic of said integrated viewing station (30i), and provided to perform the steps of:

        - receiving said reduced 3D file (R_TAC_3D) transmitted by said second processing station (20);
        - determining a 3D hologram graphic model (OLO_3D) of said patient image data (Data_Imm) as a function of said reduced 3D file (R_TAC_3D);
        - providing a viewer (303) for interacting with a user (U) and for representing said 3D hologram (OLO_3D) in a real viewing space (R_space),
        - calculating said mesh reduction factor (R_mesh) of said reduced 3D file (R_TAC_3D) as a function of said activation and configuration information (info_drive), thereby guaranteeing for said viewer (303) complete compatibility with said 3D hologram graphic model (OLO_3D) generated and allowing the user (U) to interact interactively with said 3D hologram graphic model (OLO_3D) generated.

2. The method according to claim 1, comprising - prior to the step of conditionally transmitting said reduced 3D file (R_TAC_3D) - the step of:

    - identifying in the local network (LAN) an integrated viewing station (30i) previously configured to receive said 3D file (TAC_3D);
    - detecting said identification, activation and configuration information (info_drive_I) comprised in said storage module (31) of said integrated viewing station (30i);
- sending said identification, activation and configuration information (info_drive_I) to determine a mesh reduction factor (R_mesh).

3. The method according to claim 2, further comprising the step of:

- determining said mesh reduction factor (R_mesh) for said identified integrated viewing station (30i) as a function of:

said identification, activation and configuration information (info_drive) received and/or said viewer (303) provided.

4. The method according to any one of the preceding claims, wherein said software processing of said data (Data_P,Data_Imm) takes place through the sub-steps of:

- receiving said output file (TAC_out);
- graphically processing said sequence of two-dimensional slices (seq_2D).

5. The method according to any one of the preceding claims, wherein said step of performing software processing comprises processing distinct 3D models provided in a combined view in said 3D file (TAC_3D), so that said overall 3D file (TAC_3D) represents various tissues of the patient (P) highlighted in different ways and, the method comprises, after the step of providing said viewer (303), a step of selecting said one or more tissues of the patient (P) from the reduced 3D file (R_TAC_3D).

6. The method according to any one of claims 1 to 5, wherein said software processing of said data (Data_P,Data_Imm) and/or the one or more tissues of the patient highlighted in different ways takes place through an application of a neural network system (AI) starting from said output file (TAC_out) generated by said first processing station (10).

7. The method according to claim 6, wherein said neural network system (AI) comprises a convolutional neural network trained to recognise, based on said output file (TAC_out), which regions correspond to different tissues and/or organs of interest.

8. The method according to claim 7, wherein said convolutional neural network produces a semantic segmentation, based on said output file (TAC_out), which assigns a specific class, such as bone, vessel, and other classes of tissues and/or organs, to every pixel identified.

9. The method according to claim 8, wherein said convolutional neural network performs:

- a training step, in which every input tomography test comprised in said output file (TAC_out) is accompanied by a corresponding reference segmentation; and
- a calibration step for the network parameters guided by stochastic gradient descent in order to minimise a measure of error, at the level of individual pixels, between the output of the network and said reference segmentation.

10. The method according to any one of the preceding claims, comprising the step performed in said integrated viewing station (30i) of:

- performing said interaction between the user (U) and the 3D hologram graphic model (OLO_3D) by implementing one or more functions from among movement, rotation and enlargement of said 3D hologram graphic model (OLO_3D) preferably selectable from a menu (100) of said integrated viewing station (30i).

11. The method according to claim 10, wherein said movement, rotation and enlargement functions comprise the actions of:

- detection of a hand (M) of said user (U);
- selection gestures with said hand (M) of said user (U);
- detection of a movement of said hand (M) in said selection status;
- detection of a movement of two hands (M) in said selection status.

12. A 3D processing system of a tomography test comprising:

a first processing station (10) provided to perform a tomography test (E_TAC) on a patient (P) and to generate a corresponding output data file (TAC_out) in a first predefined format for tomography tests (F_TAC), wherein said output file (TAC_out) comprises one or more from among, at least:

- patient identification data (Data_P);
- patient image data (Data_imm) comprising a sequence of two-dimensional slices (seq_2D) representative of an internal section of the patient (P);

a second processing station (20) comprising:

- a first receiving module (200) configured to receive said output file (TAC_out):

- a 3D calculating module (202) configured to generate a 3D file (TAC_3D) in a second predefined format for tomography tests (F2_TAC) as a function of said output file (TAC_out) received, wherein said 3D file (TAC_3D) comprises a plurality of 3D cells (3D_cell) that determine a 3D mesh-like structure (3D_mesh) of said image data of the patient (Data_Imm);
- a reduction module (203) configured to determine a mesh reduction factor (R_mesh) for said 3D file (TAC_3D), thus determining a reduced 3D file (R_TAC_3D),

wherein an algorithm for calculating the mesh reduction (ALG_R_mesh) comprises calculating the mesh reduction factor (R_mesh) starting from an estimated size (ES) of said output 3D file (TAC_3D) and the maximum size that the file can reach in order to be sustainable for the process of importing the mesh to an integrated viewing station (30i);

- a conditioned transmission module (204) configured to conditionally transmit in a local network (LAN) said reduced 3D file (R_TAC_3D);

wherein said conditioned transmission module (204) is configured to transmit in said local network (LAN) said reduced 3D file (R_TAC_3D) to said identified integrated viewing station (30i) through a data pack (data pack_I) comprising:

- an initialisation field (C_I);
- a message field (C_II), in particular comprising messages for and from the viewer to handle the sending of data:
- a size field (C_III) comprising the size of the data segment to be sent;
- a data field (C_IV) comprising the size data to be sent indicated in the size field (III);

wherein said size field (C_III) is defined as a function of said identification, activation and configuration information (info_drive) received. an integrated viewing station (30i;i=1..n) in said local network (LAN), configured to receive a reduced 3D file (R_TAC_3D) transmitted by said second processing station (20), and comprising:

- a storage module (301) comprising identification, activation and configuration information (info_drive_i) characteristic of said integrated viewing station (30i);
- a processing unit (302) configured to determine a 3D hologram graphic model (OLO_3D) of said patient image data (Data_Imm) as a function of said reduced 3D file (R_TAC_3D);
- a viewer (303) predisposed for interacting with a user (U) and configured to represent said 3D hologram (OLO_3D) in a real viewing space (R_space),

wherein said reduced 3D file (R_TAC_3D) has a mesh reduction factor (R_mesh) calculated as a function of said activation and configuration information (info_drive_i), thereby guaranteeing for said viewer (303) complete compatibility with said 3D hologram graphic model 3D (OLO_3D) generated and allowing the user (U) to interact interactively with said 3D hologram graphic model (OLO_3D) generated.

13. The system according to claim 12,

wherein said conditioned transmission module (204) comprises an identification module (204A) configured to:

- send call signals (Call_i) in the local network (LAN) for identifying an integrated viewing station (30i) previously predisposed to receive said 3D file (TAC_3D);
- detect said identification, activation and configuration information (info_drive_I) comprised in said storage module (301) of said identified integrated viewing station (30i);
- send said identification, activation and configuration information (info_drive_I) detected to said reduction module (203). AND

wherein said reduction module (203) is configured to determine said mesh reduction factor (R_mesh) for said identified integrated viewing station (30i), as a function of:

said identification, activation and configuration information (info_drive_I) received and/or
said viewer (303) provided.

14. The system according to any one of claims 12 to 13, wherein said second processing station (20) is configured to:

- receive said output file (TAC_out);
- graphically process said sequence of two-dimensional slices (seq_2D)
- generate said 3D file (TAC_3D) in said second predefined format for tomography tests (F2_TAC) as a function of said output file (TAC_out) received and of said processing of two-dimensional slices (seq_2D).

15. The system according to any one of claims 12 to 14, wherein said second processing station (20) comprises an application of a neural network system (AI) starting from said output file (TAC_out) generated by said first processing station (10) predisposed to generate said 3D file (TAC_3D) in said second predefined format for tomography tests (F2_TAC)

## Patentansprüche

1. Computerimplementiertes Verfahren zur 3D-Verarbeitung eines Tomographietests (E_TAC), das die folgenden Schritte umfasst:

Durchführen eines Tomographietests (E_TAC) an einem Patienten (P) und Erzeugen einer entsprechenden Datei von Ausgabedaten (TAC_out) in einem ersten vordefinierten Format für Tomographietests (F_TAC),
wobei die Ausgabedatei (TAC_out) eine oder mehrere mindestens unter Folgenden umfasst:

- Patientendaten (Data_P);
- Patientenbilddaten (Data_imm), die eine Sequenz von zweidimensionalen Schichten (Seq_2D) umfassen, die für einen internen Abschnitt des Patienten (P) repräsentativ sind;

Durchführen einer Softwareverarbeitung der Daten (Data_P,Data_Imm) zum Erzeugen einer 3D-Datei (TAC_3D) in einem zweiten vordefinierten Format für Tomographietests (F2_TAC) als Funktion der erzeugten Ausgabedatei (TAC_out), wobei die 3D-Datei (TAC_3D) eine Vielzahl von 3D-Zellen (3D_Cell) umfasst, die eine 3D-Mesh-ähnliche Struktur (3D_Mesh) der Bilddaten des Patienten (Data_Imm) bestimmen;

- Bestimmen eines Mesh-Reduktionsfaktors (R_mesh) für die 3D-Datei (TAC_3D), wodurch eine reduzierte 3D-Datei (R_TAC_3D) bestimmt wird;

wobei ein Algorithmus zum Berechnen der Mesh-Reduktion (ALG_R_mesh) das Berechnen des Mesh-Reduktionsfaktors (R_mesh) ausgehend von einer geschätzten Größe (ES) der 3D-Ausgabedatei (TAC_3D) und der maximalen Größe, die die Datei erreichen kann, um für den Prozess des Importierens vom Mesh in eine integrierte Betrachtungsstation (30i) nachhaltig zu sein, umfasst;

- Übertragen der reduzierten 3D-Datei (R_TAC_3DE) bedingt in einem lokalen Netzwerk (LAN);

wobei der Schritt zum Übertragen Übertragen der 3D-Datei (TAC_3D) in dem lokalen Netzwerk (LAN) an die identifizierte integrierte Betrachtungsstation (30i) durch ein Datenpaket vorsieht, umfassend:

- ein Initialisierungsfeld (C_I);
- ein Nachrichtenfeld (C_II), insbesondere umfassend Nachrichten für und vom Betrachter zur Abwicklung des Datenversands;
- ein Größenfeld (C_III), das die Größe des zu sendenden Datensegments umfasst;
- ein Datenfeld (C_IV), das die in dem zu sendenden Größenfeld (III) angegebenen Größendaten umfasst;

wobei das Größenfeld (C_III) als eine Funktion der empfangenen Identifikations-, Aktivierungs- und Konfigurationsinformationen (info_drive) definiert ist,
wobei das Verfahren ferner den Schritt umfasst, zum:
Bereitstellen einer integrierten Betrachtungsstation (30i) in dem lokalen Netzwerk (LAN), die Identifikations-, Aktivierungs- und Konfigurationsinformationen (info_drive) umfasst, die für die integrierte Betrachtungsstation (30i) charakteristisch sind, und bereitgestellt werden, um die folgenden Schritte durchzuführen:

- Empfangen der reduzierten 3D-Datei (R_TAC_3D), die von der zweiten Verarbeitungsstation (20) übertragen wird;
- Bestimmen eines 3D-Hologramm-Grafikmodells (OLO_3D) der Patientenbilddaten (Data_Imm) als Funktion der reduzierten 30-Datei (R_TAC_3D);
- Bereitstellen eines Betrachters (303) zum Interagieren mit einem Benutzer (U) und zum Darstellen des 3D-Hologramms (OLO_3D) in einem realen Betrachtungsraum (R_space),
- Berechnen des Mesh-Reduktionsfaktors (R_mesh) der reduzierten 3D-Datei (R_TAC_3D) als Funktion der Aktivierungs- und Konfigurationsinformationen (info_drive), wodurch dem Betrachter (303) eine vollständige Kompatibilität mit dem erzeugten 3D-Hologramm-Grafikmodell (OLO_3D) garantiert wird und es dem Benutzer (U) ermöglicht wird, interaktiv mit dem erzeugten 3D-Hologramm-Grafikmodell (OLO_3D) zu interagieren.

**2.** Verfahren nach Anspruch 1, umfassend - vor dem Schritt zum bedingten Übertragen der reduzierten 30-Datei (R_TAC_3D) - den Schritt:

- Identifizieren einer integrierten Betrachtungsstation (30i), die zuvor zum Empfangen der 3D-Datei (TAC_3D) konfiguriert wurde, in dem lokalen Netzwerk (LAN);
- Erfassen der Identifikations-, Aktivierungs- und Konfigurationsinformationen (info_drive_I), die in dem Speichermodul (31) der integrierten Betrachtungsstation (30i) enthalten sind;
- Senden der Identifikations-, Aktivierungs- und Konfigurationsinformationen (info_drive_I), um einen Mesh-Reduktionsfaktor (R_mesh) zu bestimmen.

**3.** Verfahren nach Anspruch 2, zudem umfassend den Schritt zum:

- Bestimmen des Mesh-Reduktionsfaktors (R_mesh) für die identifizierte integrierte Betrachtungsstation (30i) als Funktion von: den empfangenen Identifikations-, Aktivierungs- und Konfigurationsinformationen (info_drive) und/oder dem bereitgestellten Betrachter (303).

**4.** Verfahren nach einem der vorhergehenden Ansprüche, wobei die Softwareverarbeitung der Daten (Data_P,Data_Imm) durch die folgenden Teilschritte erfolgt:

- Empfangen der Ausgabedatei (TAC_out);
- grafisches Verarbeiten der Sequenz von zweidimensionalen Schichten (Seq_2D).

**5.** Verfahren nach einem der vorhergehenden Ansprüche, wobei der Schritt zum Durchführen der Softwareverarbeitung das Verarbeiten verschiedener 3D-Modelle umfasst, die in einer kombinierten Ansicht in der 3D-Datei (TAC_3D) bereitgestellt werden, so dass die Gesamt-3D-Datei (TAC_3D) unterschiedliche Gewebe des Patienten (P) darstellt, die auf unterschiedliche Weise hervorgehoben werden, und das Verfahren nach dem Schritt zum Bereitstellen des Betrachters (303) einen Schritt zum Auswählen des einen oder der mehreren Gewebe des Patienten (P) aus der reduzierten 3D-Datei (R_TAC_3D) umfasst.

**6.** Verfahren nach einem der Ansprüche 1 bis 5, wobei die Softwareverarbeitung der Daten (Data_P,Data_Imm) und/oder des einen oder der mehreren Gewebe des Patienten, die auf unterschiedliche Weisen hervorgehobenen wurden, durch Anwendung eines neuronalen Netzwerksystems (AI) ausgehend von der von der ersten Verarbeitungsstation (10) erzeugten Ausgabedatei (TAC_out) erfolgt.

**7.** Verfahren nach Anspruch 6, wobei das neuronale Netzwerksystem (AI) ein neuronales Faltungsnetzwerk umfasst, das darauf trainiert ist, basierend auf der Ausgabedatei (TAC_out) zu erkennen, welche Regionen unterschiedlichen Geweben und/oder Organen von Interesse entsprechen.

**8.** Verfahren nach Anspruch 7, wobei das neuronale Faltungsnetzwerk basierend auf der Ausgabedatei (TAC_out) eine semantische Segmentierung produziert, die jedem identifizierten Pixel eine bestimmte Klasse, wie etwa Knochen, Gefäß und andere Klassen von Geweben und/oder Organen, zuordnet.

**9.** Verfahren nach Anspruch 8, wobei das neuronale Faltungsnetzwerk durchführt:

- einen Trainingsschritt, bei dem jeder in der Ausgabedatei (TAC_out) enthaltene Eingangstomographietest von einer entsprechenden Referenzsegmentierung begleitet wird; und
- einen Kalibrierungsschritt für die Netzwerkparameter, der durch stochastischen Gradientenabstieg geführt wird, um ein Fehlermaß auf der Ebene einzelner Pixel zwischen dem Ausgang des Netzwerks und der Referenzsegmentierung zu minimieren.

**10.** Verfahren nach einem der vorhergehenden Ansprüche, umfassend den in der integrierten Betrachtungsstation (30i) durchgeführten Schritt zum:

- Durchführen der Interaktion zwischen dem Benutzer (U) und dem 3D-Hologramm-Grafikmodell (OLO_3D) durch Implementieren einer oder mehrerer Funktionen unter Bewegung, Drehung und Vergrößerung des 3D-Hologramm-Grafikmodells (OLO_3D), das vorzugsweise aus einem Menü (100) der integrierten Betrachtungsstation (30i) auswählbar ist.

**11.** Verfahren nach Anspruch 10, wobei die Bewegungs-, Rotations- und Vergrößerungsfunktionen die folgenden Aktionen umfassen:

- Erfassung einer Hand (M) des Benutzers (U);
- Auswahl von Gesten mit der Hand (M) des Benutzers (U);
- Erfassung einer Bewegung der Hand (M) im Auswahlstatus;
- Erfassung einer Bewegung von zwei Händen (M) in dem Auswahlstatus.

**12.** 3D-Verarbeitungssystem eines Tomographietests, umfassend:

eine erste Verarbeitungsstation (10), die bereitgestellt ist, um einen Tomographietest (E_TAC) an einem Patienten (P) durchzuführen und eine entsprechende Ausgabedatendatei (TAC_out) in einem ersten vordefinierten Format für Tomographietests (F_TAC) zu erzeugen,

wobei die Ausgabedatei (TAC_out) eine oder mehrere mindestens unter Folgenden umfasst:

- Patientenidentifikationsdaten (Data_P);
- Patientenbilddaten (Data_imm), die eine Sequenz von zweidimensionalen Schichten (Seq_2D) umfassen, die für einen internen Abschnitt des Patienten (P) repräsentativ sind;

eine zweite Verarbeitungsstation (20), umfassend:

- ein erstes Empfangsmodul (200), das konfiguriert ist, um die Ausgabedatei (TAC_out) zu empfangen:
- ein 3D-Berechnungsmodul (202), das konfiguriert ist, um eine 3D-Datei (TAC_3D) in einem zweiten vordefinierten Format für Tomographietests (F2_TAC) als Funktion der empfangenen Ausgabedatei (TAC_out) zu erzeugen, wobei die 3D-Datei (TAC_3D) eine Vielzahl von 3D-Zellen (3D_Cell) umfasst, die eine 3D-Mesh-ähnliche Struktur (3D_Mesh) der Bilddaten des Patienten (Data_Imm) bestimmen;
- ein Reduktionsmodul (203), das konfiguriert ist, um einen Mesh-Reduktionsfaktor (R_mesh) für die 3D-Datei (TAC_3D) zu bestimmen, wodurch eine reduzierte 3D-Datei (R_TAC_3D) bestimmt wird,

wobei ein Algorithmus zum Berechnen der Mesh-Reduktion (ALG_R_mesh) das Berechnen des Mesh-Reduktionsfaktors (R_mesh) ausgehend von einer geschätzten Größe (ES) der 3D-Ausgabedatei (TAC_3D) und der maximalen Größe, die die Datei erreichen kann, um für den Prozess des Importierens vom Mesh in eine integrierte Betrachtungsstation (30i) nachhaltig zu sein, umfasst;

- ein Modul für die bedingte Übertragung (204), das dazu konfiguriert ist, die reduzierte 3D-Datei (R_TAC_3D) bedingt in einem lokalen Netzwerk (LAN) zu übertragen;

wobei das Modul für die bedingte Übertragung (204) dazu konfiguriert ist, die reduzierte 3D-Datei (R_TAC_3D) in dem lokalen Netzwerk (LAN) an die identifizierte integrierte Betrachtungsstation (30i) über ein Datenpaket (data pack_I) zu übertragen, umfassend:

- ein Initialisierungsfeld (C_I);
- ein Nachrichtenfeld (C_II), insbesondere umfassend Nachrichten für und vom Betrachter zur Abwicklung des Datenversands;
- ein Größenfeld (C_III), das die Größe des zu sendenden Datensegments umfasst;
- ein Datenfeld (C_IV), das die in dem Größenfeld (III) angegebenen zu sendenden Größendaten umfasst;

wobei das Größenfeld (C_III) als eine Funktion der empfangenen Identifikations-, Aktivierungs- und Konfigurationsinformationen (info_drive) definiert ist, eine integrierte Betrachtungsstation (30i;i=1 ..n) in dem lokalen Netzwerk (LAN), die konfiguriert ist, um eine reduzierte 3D-Datei (R_TAC_3D) zu empfangen, die von der zweiten Verarbeitungsstation (20) übertragen wird, und umfassend:

- ein Speichermodul (301), das Identifikations-, Aktivierungs- und Konfigurationsinformationen (info_drive_i) umfasst, die für die integrierte Betrachtungsstation (30i) charakteristisch sind;
- eine Verarbeitungseinheit (302), die konfiguriert ist, um ein 3D-Hologramm-Grafikmodell (OLO_3D) der Patientenbilddaten (Data_Imm) als Funktion der reduzierten 3D-Datei (R_TAC_3D) zu bestimmen;
- einen Betrachter (303), der zum Interagieren mit einem Benutzer (U) eingerichtet und konfiguriert ist, um das 3D-Hologramm (OLO_3D) in einem realen Betrachtungsraum (R_space) darzustellen,

wobei die reduzierte 3D-Datei (R_TAC_3D) einen Mesh-Reduktionsfaktor (R_mesh) aufweist, der als Funktion der Aktivierungs- und Konfigurationsinformationen (info_drive_i) berechnet wird, wodurch dem Betrachter (303) eine vollständige Kompatibilität mit dem erzeugten 3D-Hologramm-Grafikmodell 3D (OLO_3D) garantiert wird und es dem Benutzer (U) ermöglicht wird, interaktiv mit dem erzeugten 3D-Hologramm-Grafikmodell (OLO_3D) zu interagieren.

13. System nach Anspruch 12,

wobei das Modul für die bedingte Übertragung (204) ein Identifikationsmodul (204A) umfasst, das konfiguriert ist, zum:

- Senden von Rufsignalen (Call_i) im lokalen Netzwerk (LAN) zum Identifizieren einer integrierten Betrachtungsstation (30i), die zuvor zum Empfangen der 3D-Datei (TAC_3D) eingerichtet war;
- Erfassen der Identifikations-, Aktivierungs- und Konfigurationsinformationen (info_drive_I), die in dem Speichermodul (301) der identifizierten integrierten Betrachtungsstation (30i) enthalten sind;
- Senden der erfassten Identifikations-, Aktivierungs- und Konfigurationsinformationen (info_drive_I) an das Reduktionsmodul (203),
UND

wobei das Reduktionsmodul (203) konfiguriert ist, um den Mesh-Reduktionsfaktor (R_mesh) für die identifizierte integrierte Betrachtungsstation (30i) als eine Funktion von:
den empfangenen Identifikations-, Aktivierungs- und Konfigurationsinformationen (info_drive_I) und/oder dem bereitgestellten Betrachter (303).

14. System nach einem der Ansprüche 12 bis 13, wobei die zweite Verarbeitungsstation (20) konfiguriert ist, zum:

- Empfangen der Ausgabedatei (TAC_out);
- grafisches Verarbeiten der Sequenz von zweidimensionalen Schichten (Seq_2D)
- Erzeugen der 3D-Datei (TAC_3D) in dem zweiten vordefinierten Format für Tomographietests (F2_TAC) als Funktion der empfangenen Ausgabedatei (TAC_out) und der Verarbeitung von zweidimensionalen Schichten (Seq_2D).

15. System nach einem der Ansprüche 12 bis 14, wobei die zweite Verarbeitungsstation (20) eine Anwendung eines neuronalen Netzwerksystems (AI) ausgehend von der von der ersten Verarbeitungsstation (10) erzeugten Ausgabedatei (TAC_out) umfasst, die dazu eingerichtet ist, die 3D-Datei (TAC_3D) in dem zweiten vordefinierten Format für Tomographietests (F2_TAC) zu erzeugen.

## Revendications

1. Procédé exécuté par ordinateur pour le traitement 3D d'un examen tomographique (E_TAC), comprenant les étapes suivantes :

effectuer un examen tomographique (E_TAC) sur un patient (P) et générer un fichier correspondant de données de sortie (TAC_out) dans un premier format prédéfini pour des examens tomographiques (F_TAC), dans lequel ledit fichier de sortie (TAC_out) comprend une ou plusieurs données parmi au moins :

- des données du patient (Data_P) ;
- des données d'image du patient (Data_imm), comprenant une séquence de coupes bidimensionnelles (seq_2D) représentatives d'une section interne du patient (P) ;

effectuer un traitement logiciel desdites données (Data_P,Data_Imm) pour générer un fichier 3D (TAC_3D) dans un second format prédéfini pour des examens tomographiques (F2_TAC) en fonction dudit fichier de sortie (TAC_out) généré, dans lequel ledit fichier 3D (TAC_3D) comprend une pluralité de cellules 3D (3D_cell) qui déterminent une structure 3D en quadrillé (3D_mesh) desdites données d'image du patient (Data_Imm) ;

- déterminer un facteur de réduction du quadrillé (R_mesh) pour ledit fichier 3D (TAC_3D), déterminant ainsi un fichier 3D réduit (R_TAC_3D) ;

dans lequel un algorithme de calcul de la réduction du quadrillé (ALG_R_mesh) comprend le calcul du facteur de réduction du quadrillé (R_mesh) à partir d'une taille estimée (ES) dudit fichier 3D de sortie (TAC_3D) et de la taille maximale que le fichier peut atteindre afin d'être soutenable pour le processus d'importation du quadrillé vers un poste de visualisation intégré (30i) ;

- transmettre ledit fichier 3D réduit (R_TAC_3DE) de manière conditionnelle dans un réseau local (LAN) ;

dans lequel ladite étape de transmission prévoit transmettre dans ledit réseau local (LAN) ledit fichier 3D (TAC_3D) audit poste de visualisation intégré identifié (30i) par l'intermédiaire d'un paquet de données comprenant :

- un champ d'initialisation (C_I) ;
- un champ de message (C_II), comprenant notamment des messages pour le dispositif de visualisation et de la part de ce dernier pour gérer l'envoi de données ;
- un champ de dimension (C_III), comprenant la dimension du segment de données à envoyer ;
- un champ de données (C_IV), comprenant les données de dimension indiquées dans le champ de dimension (III) à envoyer ;

dans lequel ledit champ de dimension (C_III) est défini en fonction desdites informations d'identification, d'activation et de configuration (info_drive) reçues,

dans lequel le procédé comprend en outre l'étape consistant à :

prévoir, dans ledit réseau local (LAN), un poste de visualisation intégré (30i) comprenant des informations d'identification, d'activation et de configuration (info_drive) caractéristiques dudit poste de visualisation intégré (30i), et prévu pour effectuer les étapes suivantes :

- recevoir ledit fichier 3D réduit (R_TAC_3D) transmis par ledit second poste de traitement (20) ;
- déterminer un modèle graphique d'hologramme 3D (OLO_3D) desdites données d'image du patient (Data_Imm) en fonction dudit fichier réduit (R_TAC_3D) ;
- prévoir un dispositif de visualisation (303) pour interagir avec un utilisateur (U) et pour représenter ledit hologramme 3D (OLO_3D) dans un espace de visualisation réel (R_space),
- calculer ledit facteur de réduction du quadrillé (R_mesh) dudit fichier 3D réduit (R_TAC_3D) en fonction desdites informations d'activation et de configuration (info_drive), garantissant ainsi audit dispositif de visualisation (303) une compatibilité totale avec ledit modèle graphique d'hologramme 3D (OLO_3D) généré et permettant à l'utilisateur (U) d'interagir interactivement avec ledit modèle graphique d'hologramme 3D (OLO_3D) généré.

2. Procédé selon la revendication 1, comprenant - avant l'étape de transmission conditionnelle dudit fichier 3D réduit (R_TAC_3D) - l'étape consistant à :

- identifier dans le réseau local (LAN) un poste de visualisation intégré (30i) préalablement configurée pour recevoir ledit fichier 3D (TAC_3D) ;
- détecter lesdites informations d'identification, d'activation et de configuration (info_drive_I) comprises dans ledit module de stockage (31) dudit poste de visualisation intégré (30i) ;
- envoyer lesdites informations d'identification, d'activation et de configuration (info_drive_I) pour déterminer un facteur de réduction du quadrillé (R_mesh) .

3. Procédé selon la revendication 2, comprenant de plus les étapes de :

- déterminer ledit facteur de réduction du quadrillé (R_mesh) pour ledit poste de visualisation intégré identifié (30i) en fonction :

desdites informations d'identification, d'activation et de configuration (info_drive) reçues et/ou dudit dispositif de visualisation (303) prévu.

4. Procédé selon l'une quelconque des revendications précédentes, dans lequel ledit traitement logiciel desdites données (Data_P,Data_Imm) s'effectue par l'intermédiaire des sous-étapes suivantes :

- recevoir ledit fichier de sortie (TAC_out) ;
- traiter graphiquement ladite séquence de coupes bidimensionnelles (seq_2D).

5. Procédé selon l'une quelconque des revendications précédentes, dans lequel l'étape consistant à effectuer le traitement logiciel comprend le traitement de modèles 3D distincts fournis dans une vue combinée dans ledit fichier 3D (TAC_3D), de sorte que le fichier 3D (TAC_3D) global représente divers tissus du patient (P) mis en évidence de différentes manières, et

le procédé comprend, après l'étape consistant à prévoir ledit dispositif de visualisation (303), une étape consistant à sélectionner ledit ou lesdits tissus du patient (P) à partir du fichier 3D réduit (R_TAC_3D).

6. Procédé selon l'une quelconque des revendications 1 à 5, dans lequel ledit traitement logiciel desdites données (Data_P,Data_Imm) et/ou du ou des tissus du patient mis en évidence de différentes manières s'effectue par une application d'un système de réseau de neurones (AI) à partir dudit fichier de sortie (TAC_out) généré par ledit premier poste de traitement (10).

7. Procédé selon la revendication 6, dans lequel ledit système de réseau de neurones (AI) comprend un réseau de neurones convolutif entraîné à reconnaître, sur la base dudit fichier de sortie (TAC_out), les régions correspondant aux différents tissus et/ou organes d'intérêt.

8. Procédé selon la revendication 7, dans lequel ledit réseau de neurones convolutif produit une segmentation sémantique, sur la base dudit fichier de sortie (TAC_out), qui attribue une classe spécifique, telle que os, vaisseau, et d'autres classes de tissus et/ou d'organes, à chaque pixel identifié.

9. Procédé selon la revendication 8, dans lequel le réseau de neurones convolutif effectue :

- une étape d'apprentissage, dans laquelle chaque examen tomographique d'entrée compris dans ledit fichier de sortie (TAC_out) est accompagné d'une segmentation de référence correspondante ; et

- une étape d'étalonnage des paramètres du réseau guidée par une descente de gradient stochastique afin de minimiser une mesure d'erreur, au niveau des pixels individuels, entre la sortie du réseau et ladite segmentation de référence.

10. Procédé selon l'une quelconque des revendications précédentes, comprenant l'étape exécutée dans ledit poste de visualisation intégré (30i) consistant à :

- effectuer ladite interaction entre l'utilisateur (U) et le modèle graphique d'hologramme 3D (OLO_3D) en mettant en œuvre une ou plusieurs fonctions parmi le mouvement, la rotation et l'agrandissement dudit modèle graphique d'hologramme 3D (OLO_3D) pouvant être sélectionnées de préférence à partir d'un menu (100) dudit poste de visualisation intégré (30i).

11. Procédé selon la revendication 10, dans lequel lesdites fonctions de mouvement, de rotation et d'agrandissement comprennent les actions suivantes :

- détection d'une main (M) dudit utilisateur (U) ;
- sélection de gestes avec ladite main (M) dudit utilisateur (U) ;
- détection d'un mouvement de ladite main (M) dans ledit état de sélection ;
- détection d'un mouvement de deux mains (M) dans ledit état de sélection.

12. Système de traitement 3D d'un examen tomographique, comprenant :

un premier poste de traitement (10) prévu pour effectuer un examen tomographique (E_TAC) sur un patient (P) et générer un fichier de données de sortie correspondant (TAC_out) dans un premier format prédéfini pour des examens tomographiques (F_TAC),
dans lequel ledit fichier de sortie (TAC_out) comprend une ou plusieurs données parmi, au moins :

- des données d'identification du patient (Data_P) ;
- des données d'image du patient (Data_imm), comprenant une séquence de coupes bidimensionnelles (seq_2D) représentatives d'une section interne du patient (P) ;

un second poste de traitement (20), comprenant :

- un premier module de réception (200) configuré pour recevoir ledit fichier de sortie (TAC_out) ;
- un module de calcul 3D (202) configuré pour générer un fichier 3D (TAC_3D) dans un second format prédéfini pour des examens tomographiques (F2_TAC) en fonction dudit fichier de sortie (TAC_out) reçu, dans lequel ledit fichier 3D (TAC_3D) comprend une pluralité de cellules 3D (3D_cell) qui déterminent une structure 3D en quadrillé (3D_mesh) desdites données d'image du patient (Data_Imm) ;
- un module de réduction (203) configuré pour déterminer un facteur de réduction du quadrillé (R_mesh) pour ledit fichier 3D (TAC_3D), déterminant ainsi un fichier 3D réduit (R_TAC_3D),

dans lequel un algorithme de calcul de la réduction du quadrillé (ALG_R_mesh) comprend le calcul du facteur de réduction du quadrillé (R_mesh) à partir d'une taille estimée (ES) dudit fichier 3D de sortie (TAC_3D) et de la taille maximale que le fichier peut atteindre afin d'être soutenable pour le processus d'importation du quadrillé vers un poste de visualisation intégré (30i) ;

- un module de transmission conditionnée (204) configuré pour transmettre conditionnellement dans un réseau local (LAN) ledit fichier 3D réduit (R_TAC_3D) ;

dans lequel ledit module de transmission conditionnée (204) est configuré pour transmettre dans ledit réseau local (LAN) ledit fichier 3D réduit (R_TAC_3D) audit poste de visualisation intégré identifié (30i) par l'intermédiaire d'un ensemble de données (data pack_I) comprenant :

- un champ d'initialisation (C_I) ;
- un champ de message (C_II), comprenant notamment des messages pour le dispositif de visualisation et de la part de ce dernier pour gérer l'envoi de données ;
- un champ de dimension (C_III), comprenant la dimension du segment de données à envoyer ;
- un champ de données (C_IV), comprenant les données de dimension à envoyer indiquées dans le champ de dimension (III) ;

dans lequel ledit champ de dimension (C_III) est défini en fonction desdites informations d'identification, d'activation et de configuration (info_drive) reçues,

un poste de visualisation intégré (30i ; i = 1 ..n) dans ledit réseau local (LAN), configuré pour recevoir un fichier 3D réduit (R_TAC_3D) transmis par ledit second poste de traitement (20), et comprenant :

- un module de stockage (301), comprenant des informations d'identification, d'activation et de configuration (info_drive_i) caractéristiques dudit poste de visualisation intégré (30i) ;
- une unité de traitement (302) configurée pour déterminer un modèle graphique d'hologramme 3D (OLO_3D) desdites données d'image du patient (Data_Imm) en fonction dudit fichier 3D réduit (R_TAC_3D) ;
- un dispositif de visualisation (303) prédisposé à interagir avec un utilisateur (U) et configuré pour représenter ledit hologramme 3D (OLO_3D) dans un espace de visualisation réel (R_space),

dans lequel ledit fichier 3D réduit (R_TAC_3D) comporte un facteur de réduction du quadrillé (R_mesh) calculé en fonction desdites informations d'activation et de configuration (info_drive_i), garantissant ainsi audit dispositif de visualisation (303) une compatibilité totale avec ledit modèle graphique d'hologramme 3D (OLO_3D) généré et permettant à l'utilisateur (U) d'interagir interactivement avec ledit modèle graphique d'hologramme 3D (OLO_3D) généré.

13. Système selon la revendication 12,

dans lequel ledit module de transmission conditionnée (204) comprend un module d'identification (204A) configuré pour :

- envoyer des signaux d'appel (Call_i) dans le réseau local (LAN) pour identifier un poste de visualisation intégré (30i) préalablement prédisposé à recevoir ledit fichier 3D (TAC_3D) ;
- détecter lesdites informations d'identification, d'activation et de configuration (info_drive_I) comprises dans ledit module de stockage (301) dudit poste de visualisation intégré identifié (30i) ;
- envoyer lesdites informations d'identification, d'activation et de configuration (info_drive_I) détectées audit module de réduction (203),
ET

dans lequel ledit module de réduction (203) est configuré pour déterminer ledit facteur de réduction du quadrillé (R_mesh) pour ledit poste de visualisation intégré identifié (30i) en fonction : desdites informations d'identification, d'activation et de configuration (info_drive_I) reçues et/ou dudit dispositif de visualisation (303) prévu.

14. Système selon l'une quelconque des revendications 12 à 13, dans lequel le second poste de traitement (20) est configuré pour :

- recevoir ledit fichier de sortie (TAC_out) ;
- traiter graphiquement ladite séquence de coupes bidimensionnelles (seq_2D) ;
- générer ledit fichier 3D (TAC_3D) dans ledit second format prédéfini pour des examens tomographiques (F2_TAC) en fonction dudit fichier de sortie (TAC_out) reçu et dudit traitement des coupes bidimensionnelles (seq_2D).

15. Système selon l'une quelconque des revendications 12 à 14, dans lequel ledit second poste de traitement (20) comprend une application d'un système de réseau de neurones (AI) à partir dudit fichier de sortie (TAC_out) généré par ledit premier poste de traitement (10) prédisposé à générer ledit fichier 3D (TAC_3D) dans ledit second format prédéfini pour des examens tomographiques (F2_TAC) .

Fig.1

DATA_PACK_i

| C_I | C_II | C_III | C_IV |
|-----|------|-------|------|

Fig.2

EP 3 939 051 B1

Case in which the file is larger than the maximum length of the data segment

Case in which the file is smaller than the maximum length of the data segment

Fig.3

Fig.4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2013044856 A1
- US 2018165867 A1

- CN 1000000

### Non-patent literature cited in the description

- Holographic Interface for three-dimensional Visualization of MRI on HoloLens: A Prototype Platform for MRI Guided Neurosurgeries. **MORALES MOJICA CRISTINA MARIE et al.** 2017 IEEE 17TH INTERNATIONAL CONFERENCE ON BIOINFORMATICS AND BIOENGINEERING (BIBE). IEEE, 23 October 2017